# EUROPEAN PATENT APPLICATION

(11) **EP 3 494 894 A1**
(43) Date of publication of application: **12.06.2019**
(21) Application number: 17205388.6
(22) Date of filing: 05.12.2017
(51) Int. Cl.: A61B 8/06, A61B 5/026

(54) **SMART COMPRESSION SLEEVE**

(71) Applicant: Koninklijke Philips N.V., 5656 AE Eindhoven (NL)
(72) Inventor: STEHLE, Thomas Heiko, 5656 AE Eindhoven (NL); WEBER, Frank Michael, 5656 AE Eindhoven (NL); WAECHTER-STEHLE, Irina, 5656 AE Eindhoven (NL); BUERGER, Christian, 5656 AE Eindhoven (NL)
(74) Representative: de Haan, Poul Erik

(57) **Abstract**

A wearable article (10) is disclosed comprising a compression sleeve (15) for compressing a region of a limb (1) of its wearer. The compression sleeve comprises at least one annular array (20, 20') of ultrasound transducer elements (21, 21') distributed along a circumference of the compression sleeve. The wearable article further comprises a controller (30) communicatively coupled to said array and arranged to operate the at least one annular array of ultrasound transducer elements in a first mode of operation in order to identify at least one ultrasound transducer element capable of detecting a blood flow through a blood vessel (3) within said limb; and operate the at least one identified ultrasound transducer element in a second mode of operation to monitor said blood flow.

## Description

### FIELD OF THE INVENTION

The present invention relates to a wearable article comprising a compression sleeve for compressing a region of a limb of its wearer, the compression sleeve comprising an ultrasound transducer for monitoring blood flow through the limb using Doppler ultrasound.

### BACKGROUND OF THE INVENTION

Deep vein thrombosis (DVT) is a well-known condition that can occur in people that are stationary for prolonged periods of time, such as bed ridden patients, frequent long haul travellers and so on. DVT is a potentially life-threatening condition, in particular when a pulmonary embolism develops from it, which can cause heart attacks and strokes. The risk of DVT and subsequent complications developing can be reduced by wearing a compression sleeve, e.g. a compression sock or stocking, as such conditions typically develop in the lower leg.

Ideally, the wearing of such a compression sleeve should be combined with periodic exercising in order to further lower this risk. In particular, where there are signs of reduced blood flow through the veins in the lower leg, this can be an indication of the onset of DVT or at least of an increased risk of such an onset, at which point in time the person wearing the compression sleeve around his or her lower leg should perform such exercises in order to improve blood circulation.

Solutions exist in which a compression sleeve is fitted with a Doppler ultrasound sensor to monitor blood flow through the lower leg. For example, in US patent US 5,843,007, an apparatus and method for periodically applying a pressure waveform to a limb are disclosed. The apparatus comprises a sleeve means adapted to position onto a limb and apply a pressure to the limb near a pressure corresponding to a sleeve pressure signal, pressure transducing means for producing an applied pressure signal indicative of the pressure applied to the limb by the sleeve means and waveform register means for producing a reference pressure waveform signal indicative of a reference pressure waveform during a predetermined cycle time period. The amplitude of the reference pressure waveform signal at any instant within the cycle time period is indicative of the amplitude of the reference pressure waveform at the instant and the shape of the reference pressure waveform during a predetermined time interval within the cycle time period is adapted to augment the flow of venous blood into the limb proximal to the sleeve means from the limb beneath the sleeve means during the interval. The apparatus further comprises pressure waveform application means responsive to the applied pressure signal and the reference pressure waveform signal and operable by producing the sleeve pressure signal to maintain the difference between the pressure indicated by the applied pressure signal and the pressure indicated by the reference pressure waveform signal at less than a predetermined pressure difference at any instant within the cycle time period.

In this apparatus, Doppler ultrasound sensor is used to measure venous blood flow velocity in order to gauge the effectiveness of the therapy administered with the sleeve means. However, a problem associated with the use of a single Doppler ultrasound sensor is that is not guaranteed to acquire a reliable blood flow signal, for example when the sensor is not located near enough to a vein. This can be overcome by adding additional sensors to the apparatus, but this introduces the problem of cross-talk between such sensors, in particularly in a pulse wave Doppler mode in which each sensor sequentially operates in a transmission and a reception mode. In such a scenario, a sensor in a reception mode may receive a pulse echo of a pulse transmitted by another sensor, which can lead to a misinterpretation of the pulse echo. This in turn may be overcome by operating one sensor at a time, but this has the drawback that the data acquisition period becomes excessively long as each sensor typically needs to implement multiple Doppler pulse wave cycles in which the gating between the transmission and reception modes is systematically varied to determine the exact location of the vein to be monitored.

### SUMMARY OF THE INVENTION

The present invention seeks to provide a wearable article comprising a compression sleeve for compressing a region of a limb of its wearer comprising a plurality of ultrasound transducers for monitoring blood flow through the limb using Doppler ultrasound that can reliably acquire such blood flow velocity data in a relatively short period of time.

According to an aspect, there is provided a wearable article comprising a compression sleeve for compressing a region of a limb of its wearer, the compression sleeve comprising at least one annular array of ultrasound transducer elements distributed along a circumference of the compression sleeve, the wearable article further comprising a controller communicatively coupled to said array and arranged to operate the at least one annular array of ultrasound transducer elements in a first mode of operation in order to identify at least one ultrasound transducer element capable of detecting a blood flow through a blood vessel within said limb; and operate the at least one identified ultrasound transducer element in a second mode of operation to monitor said blood flow.

The present invention is based on the realization that the ultrasound transducer elements of such an annular array, i.e. an array around the circumference of the limb when the wearable article is worn by its wearer, can be operated in a first mode of operation in which only qualitative data pertaining to the blood flow through the limb is acquired from which ultrasound transducer elements can be identified that are capable of collecting such blood flow information due to their vicinity to a blood vessel such as an artery or vein in the limb. Subsequently, only those ultrasound transducer elements capable of collecting such blood flow information are operated in a second mode of operation in which quantitative data pertaining to the blood flow through the limb is acquired, thereby reducing the number of ultrasound transducer elements used in the second mode of operation and reducing the duration of the acquisition period as a result. In this manner, it is ensured that the blood flow monitoring is performed with one or more ultrasound transducer elements in acoustic contact with a blood vessel through the limb whilst at the same time limiting the acquisition time of such blood flow monitoring data.

Preferably the controller is adapted to in the first mode of operation to a first subset of the ultrasound transducer elements of said at least one annular array in a continuous wave Doppler transmission mode and simultaneously operate a second subset of the ultrasound transducer elements of said at least one annular array in a reception mode. This has the advantage that by choosing an appropriate spatial arrangement of the ultrasound transducer elements in the first subset and second subset respectively, the echoes received by ultrasound transducer elements in the second subset can be used to identify the ultrasound transducer elements in the first subset that are located within acoustic range of a blood vessel within the limb.

As an example of such an appropriate spatial arrangement, each ultrasound transducer element in the first subset is located in between a pair of ultrasound transducer elements in the second subset such that the ultrasound transducer element in the first subset that is within acoustic range of such a blood vessel can be identified by its neighboring pair of ultrasound transducer elements in reception mode both receiving an echo signal indicative of the continuous wave generated by the ultrasound transducer element of the first upset having interacted with a blood flow through the limb.

The controller further may be arranged to interchange the first subset and the second subset upon completion of the continuous wave Doppler transmission mode and repeat the first mode of operation. In this manner, ultrasound transmitters become ultrasound receivers and vice versa, which increases the likelihood of an optimally positioned ultrasound transducer element relative to a blood vessel through the limb being found. This is particularly relevant where a relatively large spacing exists between neighboring ultrasound transducer elements of the array, in which case it is not guaranteed that such an optimally positioned ultrasound transducer element can be identified if only a subset of the ultrasound transducer elements of the array are operated in the transmission mode during a first mode of operation of the wearable article.

Each ultrasound transducer element may comprise a plurality of individually addressable ultrasound transducers to control a beam angle generated by the ultrasound transducer element, and wherein the controller is arranged to repeat the first mode of operation with different beam angles for at least some of the ultrasound transducer elements. This further increases the likelihood that an optimal orientation of the ultrasound transducer element relative to such a blood vessel through the limb can be found as the beam angle control may be used to focus an ultrasound beam produced by such an ultrasound transducer element onto the blood vessel of interest.

Preferably, the controller is further arranged to operate the identified at least one ultrasound transducer element in a pulse wave Doppler mode in order to monitor said blood flow in the second mode of operation, as such pulse wave Doppler ultrasound can be advantageously used to accurately locate the position of the blood vessel of interest within the limb and to monitor the blood flow velocity through such a blood vessel. To this end, the controller typically is arranged to systematically vary a delay between a transmission event and a subsequent reception event with the at least one identified ultrasound transducer element in said pulse wave Doppler mode.

In case a plurality of identified ultrasound transducer elements is identified during the first mode of operation, the controller may be arranged to operate a plurality of identified ultrasound transducer elements sequentially in said second mode of operation in order to avoid cross talk between the identified ultrasound transducer elements.

It may not always be possible to identify a vein within the limb, for example where the vein is located near the limb's axis. In such a scenario, the controller may be arranged to operate one of the at least one identified ultrasound transducer element in a first sub mode to identify a first location within the limb in which a first blood flow is directed away from the heart, operate one of the at least one identified ultrasound transducer element in a second sub mode to identify a second location within the limb proximal to the first location in which a second blood flow is directed towards the heart, and monitor the second blood flow. In this embodiment, an artery is first located, which location is used as a starting point for the identification of a vein in its vicinity, as the location of the vein typically can be found in close proximity to an artery.

In another advantageous embodiment, the at least one annular array of ultrasound transducer elements comprises a plurality of annular arrays of ultrasound transducer elements spatially distributed along the compression sleeve such that the blood flow may be measured in multiple locations along the limb, which for example may exist in pinpointing the location of the course of a reduction in the blood flow velocity through the limb, e.g. a stenosis or the like.

In addition, the controller may be arranged to interpret the respective blood flows monitored by the plurality of annular arrays of ultrasound transducer elements on a consensus or majority basis in order to reduce the number of false alarms produced by the controller of the wearable article, as such an alarm only will be raised if a majority or all of the annular arrays report an abnormal blood flow velocity through the limb.

The functionality of the wearable article may be further extended in that the controller may be further arranged to capture an ultrasound image of the blood vessel with the at least one annular array of ultrasound transducer elements, e.g. a B-mode image. Such an image may be made available to a clinician in order to diagnose or identify blood flow problems through the limb, e.g. to detect veins exhibiting low blood flow velocities adjacent to arteries having high blood flow velocities. In this embodiment, the ultrasound frequency used for acquiring such an ultrasound image may be optimized based on the depth information regarding the location of the vein of interest as retrieved in the second mode of operation in other to optimize the resolution of the ultrasound image.

The wearable article may further comprise an output device communicatively coupled to the controller for producing an output indicative of a monitoring result of the monitored blood flow. For example, the wearable article may comprise a loudspeaker, a display or the like onto which such a monitoring result, e.g. an alarm indicative of an unusually low blood flow velocity through the monitored vein, may be generated. Alternatively, the output device may be a wireless communication module for providing data indicative of the monitoring result to an external device for generating such an alarm or even processing such data on the external device.

In at least some embodiments, the wearable article is a sock or stocking although it should be understood that embodiments of the present invention are not limited thereto. It is for instance equally feasible that the wearable article is a compression sleeve, e.g. a sleeve that when worn on a lower leg of its wearer, does not have a foot portion covering the foot attached to that leg.

### BRIEF DESCRIPTION OF THE DRAWINGS

Embodiments of the invention are described in more detail and by way of non-limiting examples with reference to the accompanying drawings, wherein:
Fig. 1 schematically depicts a wearable article according to an embodiment;
Fig. 2 schematically depicts a principle of operation of such a wearable article;
Fig. 3 depicts a flowchart of operating method of such a wearable article according to an embodiment;
Fig. 4 schematically depicts an operating mode of such a wearable article according to an embodiment; and
Fig. 5 schematically depicts another operating mode of such a wearable article according to an embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

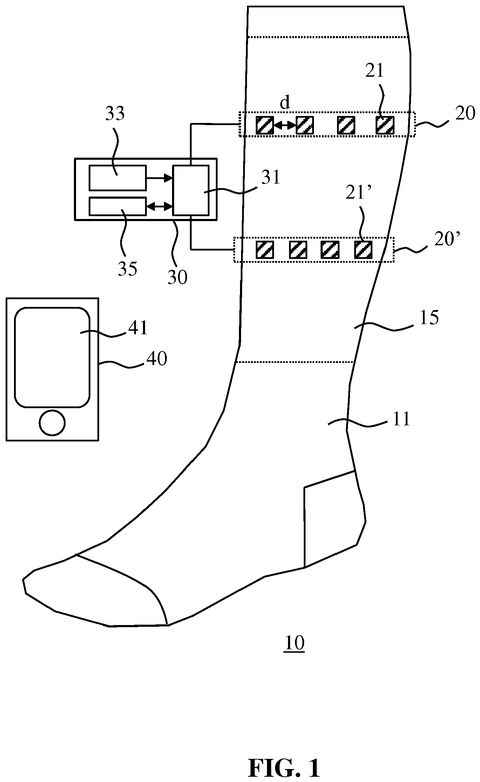

Fig. 1 schematically depicts a wearable article 10 according to an example embodiment. The wearable article 10 comprises a compression sleeve 15 for fitting around a limb of its wearer, such as a lower leg. The compression sleeve 15 may be made of any suitable material capable of applying such a compression, such as for example any natural or man-made fiber or combinations thereof. A non-limiting example of a suitable material is a fiber mix including an elastic polymer such as an elastic polyurethane (elastane). Alternatively or additionally, the wearable article 10 may comprise inflatable compartments (not shown) that may be used to massage or apply a similar therapy to the limb in order to stimulate blood circulation through the limb, such as for example disclosed in US 5,843,007. The compression sleeve 15 may be attached to a foot portion 11 of the wearable article 10 in case of the wearable article 10 being a compression sock or a compression stocking. Alternatively, the foot portion 11 may be omitted in which case the wearable article 10 may only comprise a compression sleeve 15 as the portion of the wearable article to be worn by its wearer.

Around the compression sleeve 15, at least one annular array 20 of ultrasound transducer elements 21 is present. The annular array 20 of ultrasound transducer elements 21 is arranged such that when the compression sleeve 15 is worn by its wearer around a limb, the annular array 20 of the ultrasound transducer elements 21 defines a circumference around a portion of the limb. Within the context of the present application, where reference is made to a ultrasound transducer element, it should be understood that such an element may be an ultrasound transducer chip comprising a plurality of ultrasound transducer cells that can be individually addressed for beam steering purposes as is well-known per se, such that this will not be explained in further detail for the sake of brevity only. The ultrasound transducer elements 21 may be attached to the compression sleeve 15 in any suitable manner. For example, the ultrasound transducer elements 21 may be attached to an outer surface of the compression sleeve 15 or alternatively the ultrasound transducer elements 21 may be fitted into pockets (not shown) within the compression sleeve 15, e.g. the compression sleeve 15 may be double skinned in which case the ultrasound transducer elements 21 may be affixed in between the skin layers of the compression sleeve 15. As will be understood by the skilled person, the ultrasound transducer elements 21 are typically fitted such that the ultrasound emitting surfaces of the ultrasound transducer elements 21 face the limb of the wearer of the wearable article 10 such that the ultrasound waves generated by these ultrasound transducer elements 21 may penetrate the limb.

The ultrasound transducer elements 21 within the annular array 20 preferably are equidistantly spaced by a distance d, which distance is a function of the total number of ultrasound transducer elements 21 and the diameter of the circumference as will be readily understood by the skilled person. Any suitable number of ultrasound transducer elements 21 may be present within the annular array 20. As will be explained in further detail below, selected ultrasound transducer elements 21 are used to monitor the blood flow velocity through a vein in the limb of the wearer of the wearable article 10, which selection is typically (much) smaller than the total number of ultrasound transducer elements 21 within the annular array 20. In a particular embodiment, the selection of ultrasound transducer elements 21 comprises a single ultrasound transducer element 21 only.

Any suitable type of ultrasound transducer element may be used in the one or more annular arrays such as piezoelectric or capacitive ultrasound transducer elements. In a preferred embodiment, the ultrasound transducer elements are capacitive micro-machined ultrasound transducer (CMUT) elements, which may be operable in a so-called collapse mode in order to improve the spectral bandwidth and output pressure of such transducer elements. More generally speaking, CMUT elements are preferred due to their ease of manufacture and superior output characteristics compared to piezoelectric elements such as PZT elements.

In some embodiments, the compression sleeve 15 may comprise a plurality of such annular arrays, here symbolized by the presence of the annular array 20 of ultrasound transducer elements 21 and a further annular array 20' of ultrasound transducer elements 21' by way of non-limiting example only. It should be understood that any suitable number of such annular arrays may be present on the compression sleeve 15. Where such a plurality of annular arrays is present, each annular array may be configured to independently monitor blood flow velocity through the limb around which the compression sleeve 15 is fitted. In such a scenario, the controller 30 may receive multiple blood flow velocity readings from the different annular arrays. As will be explained in more detail below, the controller 30 may be arranged to only act upon the blood flow velocity readings indicative of diminished blood flow through a monitored vein within the limb if a majority of the annular arrays report such a diminished blood flow or alternatively if all annular arrays reports such a diminished blood flow, thereby implementing majority or consensus decision-making within the controller 30. This reduces the risk that a false alarm of the detection of such a diminished blood flow is generated by the controller 30.

The controller 30 may comprise a processor arrangement 31 arranged to control the ultrasound transducer elements 21, 21' of the annular arrays 20, 20' and to receive ultrasound data captured by the ultrasound transducer elements 21, 21' of the annular arrays 20, 20'. The processor arrangement 31 may be further arranged to at least partially process the received ultrasound data or alternatively the ultrasound data may be forwarded to an external device for further processing. The controller 30 further may comprise a power supply 33, e.g. a battery, a rechargeable battery or the like although such a power supply 33 may be omitted where the controller 30 may be powered by a mains power supply.

The controller 30 may further comprise an output device 35. In a first set of embodiments, the output device 35 is a device arranged to produce a sensory output for the wearer of the wearable article 10 such as a visible or audible alarm that informs the wearer that the blood flow velocity through his or her limb has reduced, thereby encouraging the wearer to perform a set of exercises in order to stimulate the blood flow through the limb. In a second set of embodiments, the output device 35 is a wireless communication module arranged to communicate with an external device 40 comprising an external article device 41, e.g. a loudspeaker, a display screen, or the like onto which such an alarm may be generated. Any suitable wireless communication protocol such as Wi-Fi, Bluetooth, a mobile communication protocol such as GSM, UMTS, a proprietary communication protocol and so on may be used for the communication between the output device 35 and the external device 40. Alternatively, the output module 35 may be configured to communicate over a wired connection, e.g. through a cable or the like, with the external device 40.

The external device 40 is depicted as a smart phone by way of non-limiting example only. Although the external device 40 may be a smart phone or another portable device such as a smart watch or a tablet computer, which for example may facilitate a long haul traveller to keep track of the blood flow monitoring performed by the one or more annular arrays 20, 20', the external device 40 alternatively may be a central server, computer or the like, which for example may be advantageous in a medical facility such as a hospital to centrally monitor the blood flow through the limbs of multiple bed-ridden patients in the medical facility. This for example may be used to trigger medical professionals to engage with such patients for exercise purposes in order to stimulate blood circulation through the limbs upon detection upon a diminished blood flow through these limbs.

In case of the wearable article 10 comprising a power supply 33 in the form of a battery, in order to maintain battery life, the processing of the echo signals acquired with the one or more annular array(s) may be performed on the external device 40. To this end, the controller 30 may be configured to perform some pre-processing of the echo signals, e.g. down conversion or the like, in order to reduce the amount of data that needs to be communicated to the external device 40, thereby further extending battery life. However, in an alternative embodiment the processor arrangement 31 further comprises a signal processor, thereby providing a self-contained device, which may be configured to communicate minimal amounts of data, e.g. processing results or an alarm, to the external device 40, e.g. for visualization purposes.

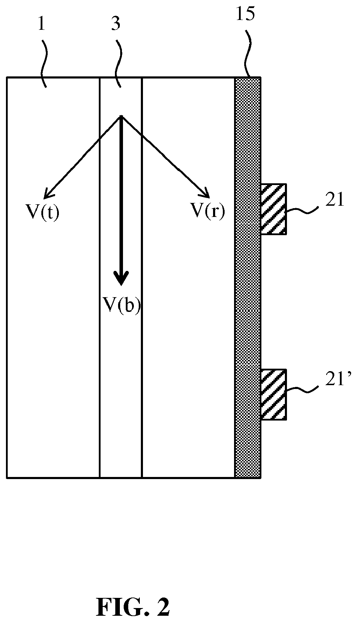

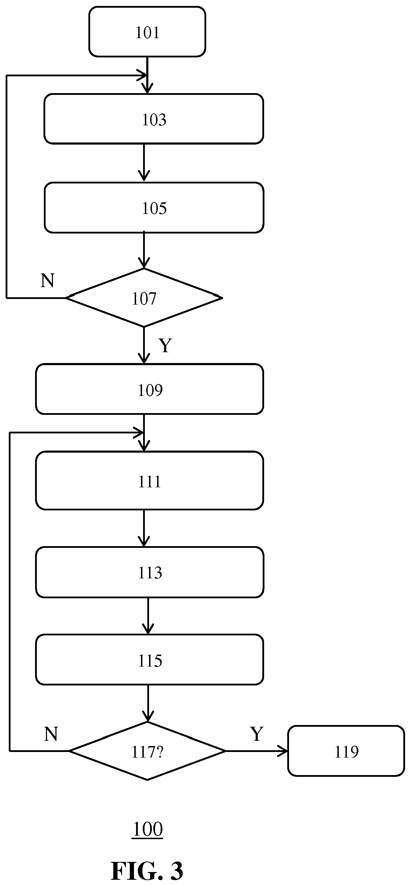

Fig. 2 schematically depicts the operating principle on which the ultrasound transducer elements 21, 21' are based. Blood flows with a velocity V(b) through a blood vessel 3, e.g. a vein, of the limb 1. This blood flow velocity V(b) has a tangential component V(t) and a radial component V(r). The radial component V(r) may be detected using the transducer elements 21, 21' in or on the compression sleeve 15 surrounding the limb 1 using Doppler ultrasound measurements under control of the controller 30. The operation of the ultrasound transducers 21, 21' in an annular array 20, 20' by the controller 30 will now be explained in further detail with the aid of Fig. 3, which depicts a flowchart of an example embodiment of an operating method 100 implemented by the controller 30.

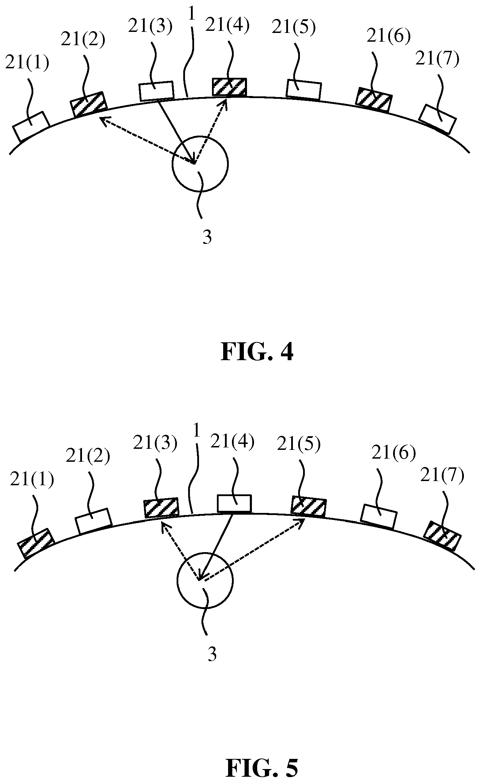

The operating method 100 starts in operation 101, e.g. by powering up the controller 30, after which the operating method 100 proceeds to a first mode of operation 103 in which for each annular array 20, 20' the ultrasound transducer elements 21, 21' are divided into two groups or subsets; a first subset containing the ultrasound transducer elements 21, 21' that will operate as ultrasound transmitters and a second subset containing the ultrasound transducer elements 21, 21' that will operate as ultrasound receivers. This is schematically depicted in Fig. 4, in which the ultrasound transducers 21(1), 21(3), 21(5) and 21(7) belong to the first subset, i.e. are operated as ultrasound transmitters and the ultrasound transducers 21(2), 21(4) and 21(6) belong to the second subset, i.e. are operated as ultrasound receivers. The compilation of the first subset and the second subset preferably is made such that an alternating pattern of ultrasound transmitters and receivers is formed around the circumference of the limb 1, that is, each ultrasound transducer element 21(1), 21(3), 21(5), 21(7) in the first subset is located in between a pair of ultrasound transducer elements 21(2), 21(4), 21(6) in the second subset, or alternatively, each ultrasound transmitter has at least one and preferably two ultrasound receivers as its immediate neighbors in the annular array 20 (or 20'). Of course, other suitable geometries of the first and second subsets may be equally contemplated.

In a particular embodiment, the controller 30 operates the first subset of ultrasound transducer elements, i.e. the ultrasound transmitters in a continuous wave Doppler mode. In such a mode, the ultrasound transmitters continuously send out pulses of ultrasound, whilst the ultrasound receivers continuously listen for the multitude of reflected frequency shifts that are reflected back by the blood flow. Continuous wave Doppler facilitates the detection of the magnitude of blood flows, without being able to detect the actual location of the blood flow, i.e. the blood vessel 3, within the limb 1. However, as schematically depicted in Fig. 4, not all ultrasound receivers, i.e. ultrasound transducer elements 21(2), 21(4) and 21(6), will receive such frequency-shifted echoes, or at least receive such frequency-shifted echoes of a certain amplitude, because not all of the ultrasound transmitters, i.e. ultrasound transducer elements 21(1), 21(3), 21(5) and 21(7) will be within acoustic range of such a blood vessel 3.

This aspect is leveraged in operation 105 in which the controller 30 evaluates which of the ultrasound receivers have received the strongest frequency-shifted echoes. This is symbolically indicated in Fig. 4 by the dashed arrows incident on ultrasound transducer elements 21(2) and 21(4), which represent the frequency-shifted echoes of the ultrasound pulses generated by the ultrasound transmitter, i.e. ultrasound transducer element 21(3) as indicated by the solid arrow. The controller 30 identifies the ultrasound receivers having received the strongest frequency-shifted echoes, and identifies the ultrasound transmitter responsible for the continuous wave Doppler pulses causing these frequency-shifted echoes from the spatial relationship between the ultrasound transmitters and the ultrasound receivers in the annular array 20 in the first mode of operation, here ultrasound transducer element 21(3) operating as an ultrasound transmitter, with the neighboring ultrasound transducer elements 21(2) and 21(4) acting as ultrasound receivers receiving the frequency-shifted echoes. In this mode of operation, the controller 30 therefore detects those ultrasound transducer elements 21, 21' that are capable of detecting a blood flow through the limb 1 of the wearer of the wearable article 10.

In a further refinement, the controller 30 may check in operation 107 if the first mode of operation needs to be repeated with a different compilation of the first and second subsets. This for example may be required where the spacing d between ultrasound transducer elements 21, 21' in the one or more annular arrays 20, 20' is large enough such that it cannot be ensured that an optimally positioned ultrasound transducer element relative to the blood vessel 3 can be found in a single cycle of the first mode of operation, e.g. a single cycle of the CW Doppler mode. If this is the case, it may be decided in operation 107 to revert back to operation 103 in which the first mode of operation of the wearable article 10 is repeated. For example, the roles of the ultrasound transmitters and ultrasound receivers may be interchanged as schematically depicted in Fig. 5 such that the first subset of ultrasound transducer elements acting as ultrasound transmitters now comprises the ultrasound transducer elements 21(2), 21(4) and 21(6), whereas the second subset of ultrasound transducer elements acting as ultrasound receivers now comprises the ultrasound transducer elements 21(1), 21(3), 21(5) and 21(7). Again, one or more ultrasound transducer elements acting as ultrasound transmitters may be identified by the controller 30 as being in acoustic range of the blood vessel 3 within the limb 1 based on the frequency-shifted echoes received by the ultrasound receivers in close vicinity to such an ultrasound transmitter, here ultrasound transducer element 21(4).

The controller may then select the ultrasound transmitter having caused the strongest frequency-shifted echoes from the multiple cycles of the first mode of operation in operation 109 to identify the ultrasound transducer element of a particular annular array to be used for the monitoring of the blood flow velocity through a vein within the limb 1. Although this procedure has been explained for a single annular array, it should be understood that the same procedure may be applied to any of the annular arrays 20, 20' on the compression sleeve 15, which procedure may be applied sequentially or simultaneously to such arrays in case of multiple annular arrays being present on the compression sleeve 15.

Next, the operating method 100 proceeds to operation 111 in which the one or more ultrasound transducer elements identified in operation 109 are operated to monitor the blood flow velocity through a vein in the limb 1, preferably using pulse wave Doppler ultrasound. In this mode of operation, the identified ultrasound transducer element(s) sends out a pulsed signal to a depth defined by the controller 30 in a transmission mode and then switches to a silent mode or a reception mode in operation 113 for a defined period of time in which the ultrasound transducer elements listens for the reflected frequency-shifted echo from that particular depth. The frequency shift scales with the velocity of the blood flow at that particular depth such that the controller 30 (or an external processor as previously explained) can calculate the blood flow velocity at that particular depth in operation 115.

As indicated by operation 117, the controller 30 may systematically vary the depth within the limb 1 to which the pulsed signal by the identified ultrasound transducer element(s) in order to accurately locate the vein within the limb 1, in which case the operating method 100 returns to operation 111 to perform another pulse wave Doppler cycle in which the pulse penetration depth has been altered, and in a delay between the transmission event and the subsequent reception event is altered accordingly. This process may be repeated until the controller 30 has accurately determined the location of the vein within the limb 1.

It is further noted that where the second mode of operation, i.e. the pulse wave Doppler ultrasound mode, is performed with more than one ultrasound transducer element 21, 21' of a particular annular array 20, 20', only one of these ultrasound transducer elements is used at the same time to avoid crosstalk between such ultrasound transducer elements. In such a case, the controller 30 may decide in operation 117 to revert back to operation 111 in which another of the ultrasound transducer elements identified in a first mode of operation is selected to perform the pulse wave Doppler ultrasound.

In a scenario where the detected blood vessel 3 is an artery in a central location of the limb 1, it may not be straightforward to identify a vein in this location for blood flow monitoring due to the low intensity of the measured blood flow signal from the blood flow through such a centrally located vein. In such a scenario, such a vein may be detected by scanning for locations within the limb 1 in a first sub mode that are characterized by blood flow directed away from the heart, e.g. blood flow through a central artery, after which in a second sub mode a region proximal to the identified blood flow is evaluated to detect a blood flow towards the heart, i.e. blood flow through a vein. This for example may be achieved using pulse wave Doppler ultrasound, in which such an adjacent region is systematically scanned using the originally identified ultrasound transducer element, e.g. by systematically altering the beam angle of this ultrasound transducer element to scan the adjacent region, or by selecting an ultrasound transducer element neighboring the originally identified ultrasound transducer element to scan such an adjacent region. In this manner, the location of such a centrally located vein can be accurately determined and the blood flow velocity through this vein monitored as explained above. Upon completion of this second mode of operation, the operating method 100 may terminate in operation 119.

In an alternative embodiment, the wearable article 10 comprises a plurality of annular arrays including a first annular array 20 of ultrasound transducer elements 21 and a second annular array 20' of ultrasound transducer elements 21' spatially separated from the first annular array 20, wherein each ultrasound transducer element 21,21' comprises a single ultrasound transducer only. In this embodiment, blood flow direction may be detected by operating all ultrasound transducer elements 21 of the first annular array 20 in a transmission mode and all ultrasound transducer elements 21' of the second annular array 20' in a reception mode. Because the signal path of the ultrasound signals is parallel to the blood flow, one could also determine flow direction in this setup.

As will be understood from the foregoing, the controller 30 or the external device 40 may use the monitored blood flow velocity of a monitored vein to generate an alarm when the monitored blood flow velocity falls below a defined threshold or is continually decreasing. As previously explained, such an alarm may be intended to trigger the wearer of the wearable article 10 to perform exercises to stimulate blood circulation through the limb 1 or to alert another person, e.g. a medical professional such as a nurse, to attend to the wearer of the wearable article 10 in order to alert the wearer to the need for performing such exercises or alternatively massage the limb of the wearer in case the wearer is physically incapable of performing such exercises, as may be the case with bed-ridden patients. Additionally or alternatively, the blood flow velocity monitoring data may be visualized on the output device such as the output device 35 or the external output device 41 such that the wearer of the wearable article 10 or anybody else monitoring this data can keep track of the blood flow velocity through the monitored vein over time.

In a further embodiment, the one or more annular arrays 20, 20' of ultrasound transducer elements 21, 21' may be arranged to acquire an ultrasound image of the target region within the limb 1, i.e. the region including the vein being monitored. Such an ultrasound image for example may be a B-mode ultrasound image. The image data may be evaluated together with the blood flow velocity monitoring data, e.g. to identify the veins having no blood flow adjacent to arteries having a high blood flow, such that problematic veins can be visualized in this manner. The parameters used for the acquisition of such an ultrasound image may be based on the depth information extracted from the aforementioned pulse wave Doppler ultrasound mode in order to optimize the resolution of the target region of interest captured in such an ultrasound image.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A wearable article (10) comprising a compression sleeve (15) for compressing a region of a limb (1) of its wearer, the compression sleeve comprising at least one annular array (20, 20') of ultrasound transducer elements (21, 21') distributed along a circumference of the compression sleeve, the wearable article further comprising a controller (30) communicatively coupled to said array and arranged to:
operate the at least one annular array of ultrasound transducer elements in a first mode of operation in order to identify at least one ultrasound transducer element capable of detecting a blood flow through a blood vessel (3) within said limb; and
operate the at least one identified ultrasound transducer element in a second mode of operation to monitor said blood flow.

2. The wearable article (10) of claim 1, wherein the controller (30) is adapted to, in said first mode of operation, operate a first subset (21(1), 21(3), 21(5), 21(7)) of the ultrasound transducer elements (21, 21') of said at least one annular array (20, 20') in a continuous wave Doppler transmission mode and simultaneously operate a second subset (21(2), 21(4), 21(6)) of the ultrasound transducer elements of said at least one annular array in a reception mode.

3. The wearable article (10) of claim 2, wherein each ultrasound transducer element (21(1), 21(3), 21(5), 21(7)) in the first subset is located in between a pair of ultrasound transducer elements (21(2), 21(4), 21(6)) in the second subset.

4. The wearable article (10) of claim 2 or 3, wherein the controller is arranged to interchange the first subset and the second subset upon completion of the continuous wave Doppler transmission mode and repeat the first mode of operation.

5. The wearable article (10) of any of claims 1-4, wherein each ultrasound transducer element (21,21') comprises a plurality of individually addressable ultrasound transducers to control a beam angle generated by the ultrasound transducer element, and wherein the controller (30) is arranged to repeat the first mode of operation with different beam angles for at least some of the ultrasound transducer elements.

6. The wearable article (10) of any of claims 1-5, wherein the controller (30) is arranged to, in said second mode of operation, operate the at least one identified ultrasound transducer element (21, 21') in a pulse wave Doppler mode in order to monitor said blood flow.

7. The wearable article (10) of claim 6, wherein the controller (30) is arranged to systematically vary a delay between a transmission event and a subsequent reception event with the at least one identified ultrasound transducer element (21, 21') in said pulse wave Doppler mode.

8. The wearable article (10) of claim 6 or 7, wherein the controller (30) is arranged to operate a plurality of identified ultrasound transducer elements (21,21') sequentially in said second mode of operation.

9. The wearable article (10) of any of claims 5-8, wherein the controller (30) is arranged to:
operate one of the at least one identified ultrasound transducer element (21, 21') in a first sub mode to identify a first location within the limb (1) in which a first blood flow is directed away from the heart;
operate one of the at least one identified ultrasound transducer element in a second sub mode to identify a second location within the limb proximal to the first location in which a second blood flow is directed towards the heart, and
monitor the second blood flow.

10. The wearable article (10) of any of claims 1-9, wherein the at least one annular array (20, 20') of ultrasound transducer elements (21, 21') comprises a plurality of annular arrays of ultrasound transducer elements spatially distributed along the compression sleeve (15).

11. The wearable article (10) of claim 10, wherein the controller (30) is arranged to interpret the respective blood flows monitored by the plurality of annular arrays (20, 20') of ultrasound transducer elements (21, 21') on a consensus or majority basis.

12. The wearable article (10) of any of claims 1-11, wherein the controller (30) is further arranged to capture an ultrasound image of the blood vessel (3) with the at least one annular array (20, 20') of ultrasound transducer elements (21, 21').

13. The wearable article (10) of any of claims 1-12, further comprising an output device (35) communicatively coupled to the controller (30) for producing an output indicative of a monitoring result of the monitored blood flow.

14. The wearable article (10) of claim 13, wherein the output device (25) is a wireless communication module.

15. The wearable article (10) of any of claims 1-14, wherein the wearable article is a sock or stocking.
